# EUROPÄISCHE PATENTANMELDUNG

(11) **EP 2 455 047 A1**
(43) Veröffentlichungstag der Anmeldung: **23.05.2012**
(21) Anmeldenummer: 10192020.5
(22) Anmeldetag: 22.11.2010
(51) Int. Cl.: A61F 13/15, B29C 33/36

(54) **Formstation zum Herstellen von Formkörpern**

(71) Anmelder: BIKOMA AG Spezialmaschinen, 56727 Mayen/Eifel (DE)
(72) Erfinder: Goebel, Bernd, 56761 Gamlen (DE)
(74) Vertreter: Nübold, Henrik

(57) **Zusammenfassung**

Eine Formstation zur Herstellung von Formkörpern mit einer Anzahl von Formwerkzeugen (4), welche Formwerkzeuge, dazu ausgebildet sind, zum Formen des Formkörpers aus mindestens einer Materialbahn mit einer Fördereinrichtung für die Materialbahn zusammenzuwirken, in welcher Formstation die Formwerkzeuge (4) zumindest während einer zum Formen des Formkörpers benötigten Einwirkzeit in einem Bearbeitungsbereich synchron zusammen mit der Materialbahn bewegbar und dabei in wenigstens einer ersten geschlossenen Werkzeugführungsstruktur (5) geführt sind, wobei die Werkzeugführungsstruktur (5) zumindest in dem Bearbeitungsbereich einen geraden Abschnitt (5.1) aufweist und dort im Wesentlichen parallel zur Materialbahn verläuft, zeichnet sich dadurch aus, dass zum Bewegen der Formwerkzeuge (4) wenigstens eine Antriebseinrichtung vorgesehen ist, welche dazu ausgebildet ist, auf wenigstens eines der Formwerkzeuge (4) einzuwirken, wobei die Formwerkzeuge (4) in der Werkzeugführungsstruktur (5) im Wesentlichen lückenlos angeordnet sind und schiebend aufeinander einwirken

## Beschreibung

Die vorliegende Erfindung betrifft eine Formstation nach dem Oberbegriff des Patentanspruchs 1 zur Herstellung von Formkörpern mit einer Anzahl von Formwerkzeugen, welche Formwerkzeuge dazu ausgebildet sind, zum Formen des Formkörpers aus mindestens einer Mit einer Fördereinrichtung für die Materialbahn zusammenzuwirken, in welcher Formstation die Formwerkzeuge zumindest während einer zum Formen des Formkörpers benötigten Einwirkzeit in einem Bearbeitungsbereich synchron zusammen mit der Materialbahn bewegbar und dabei in wenigstens einer ersten geschlossenen Werkzeugführungsstruktur geführt sind, wobei die Werkzeugführungsstruktur zumindest in dem Bearbeitungsbereich einen geraden Abschnitt aufweist und dort im Wesentlichen parallel zur Materialbahn verläuft.

Weiterhin betrifft die Erfindung eine Verwendung der erfindungsgemäßen Formstation.

Bei der Herstellung von Formkörpern, insbesondere saugfähigen Formkörpern, wie Stilleinlagen oder dergleichen, kamen in der Vergangenheit vielfach Formstationen zum Einsatz, bei denen eine Materialbahn aus dem Formkörpermaterial mittels einer Fördereinrichtung zu einem Formwerkzeug gefördert wird, welches dann das Formkörpermaterial der Materialbahn zur Herstellung des Formkörpers geeignet formt. In diesem Zusammenhang ist insbesondere bekannt, dass das Formwerkzeug derart mit der Fördereinrichtung für die Materialbahn zusammenwirkt, dass diese während einer zum Formen des Formkörpers benötigten Einwirkzeit, das heißt während das Formwerkzeug auf die Materialbahn bzw, das Formkörpermaterial einwirkt, angehalten wird, um die genannte Einwirkzeit zu erreichen. Hierbei war insbesondere als nachteilig anzusehen, dass die Materialbahn während der gesamten Einwirkzeit stillsteht, was sich negativ auf den erreichbaren Durchsatz einer derartigen Formstation auswirkt, so dass keine hohen Stückzahlen erreicht werden konnten.

Weiterhin sind aus dem Stand der Technik Formstationen mit walzenartigen Formwerkzeugen bekannt, bei denen es zwar nicht zu einem Stillstand der Materialbahn kommt, jedoch aufgrund der geometrischen Ausgestaltung einer derartigen Formstation Verzerrungen bei der Formgebung auftreten. Außerdem ist die Einwirkzeit nachteiliger Weise relativ kurz.

Des Weiteren offenbart die DE 10 2005 022 269 A1 eine Anlage und ein Verfahren zum Herstellen eines speziellen Hygleneartikels, wobei durch Relativgeschwindigkeiten von Führungseinrichtungen in Bezug auf eine Führungsgeschwindigkeit der Materialbahn eine mehrfache Faltung des Hygieneartikels, wie einer Windel, erreichbar ist.

Zur Vermeidung der vorstehend erwähnten Nachteile wurde durch die Anmelderin in der DE 10 2007 030 008 die Schaffung einer gattungsmäßen Formstation vorgeschlagen, wie sie in dem Oberbegriff des Patentanspruchs 1 definiert ist. In der genannten Formstation sind die Formwerkzeuge zumindest während einer zum Formen des Formkörpers benötigten Einwirkzeit in einem Bearbeitungsbereich synchron zusammen mit der Materialbahn bewegbar und dabei in wenigstens einer ersten geschlossenen Werkzeugführungsstruktur geführt, wobei die Werkzeugführungsstruktur zumindest in dem Bearbeitungsbereich einen geraden Abschnitt aufweist und dort im Wesentlichen Parallel zur Materialbahn verläuft. Dadurch wurden längere Einwirkzeiten und verzerrungsfreie Formgebungen bei erhöhtem Durchsatz möglich.

Um Textwiederholungen nach Möglichkeit zu vermeiden, wird im Rahmen der vorliegenden Anmeldung explizit auf die Offenbarung in der Offenlegungsschrift DE 10 2007 030 008 A1 Bezug genommen, deren gesamter Offenbarungsgehalt hiermit durch Referenzierung in die vorliegende Anmeldung aufgenommen wird.

Beim Gegenstand der DE 10 2007 030 008 A1 ist die synchrone Bewegung von Formwerkzeug und Materialbahn in dem gerade Bearbeitungsbereich mittels einer speziellen Antriebseinrichtung in Form einer Schneckenwelle realisiert. Außerhalb des Bearbeitungsbereichs erfolgt der (Rück-)Transport der Formzeuge entlang der Werkzeugführungsstruktur mittels eines doppelten Reibradantriebs gegenüber der Bewegung im Bearbeitungsbereich beschleunigt, um die erforderliche Anzahl von Formwerkzeugen möglichst gering zu halten.

Im der Praxis hat sich allerdings gezeigt, dass die konstruktive Ausführung des vorstehend beschriebenen Reibradantriebs problematisch sein kann, da einerseits ein hoher Reibwert vorhanden sein muss, um eine sichere Mitnahme und Beschleunigung der Formwerkzeuge durch die Reibräder zu gewährleisten, wobei andererseits keine übermäßige Wärmeentwicklung beim Abbremsen und Stauen der Formwerkzeuge auftreten darf. Darüber hinaus bewirken die Reibräder Materialabrieb sowie einen relativ unruhigen Betrieb der Formstation durch Wechsel von Beschleunigung und Abbremsen, wobei es zu einem Aufschwingen der Konstruktion kommen kann.

Der Erfindung liegt die Aufgabe zugrunde, eine Formstation der eingangs genannten Art dahingehend weiterzuentwickeln, dass zur Vermeidung der vorstehend skizzierten Problematik auf einen Reibradantrieb verzichtet werden kann.

Diese Aufgabe wird gelöst durch eine Formstation mit den Merkmalen des Patentanspruchs 1.

Vorteilhafte Weiterbildungen der erfindungsgemäßen Formstation sind jeweils Gegenstand von Unteransprüchen, deren Wortlaut hiermit durch ausdrückliche Bezugnahme in die Beschreibung aufgenommen wird, um unnötige Textwiederholungen zu vermeiden.

Erfindungsgemäß ist eine Formstation zur Herstellung von Formkörpern mit einer Anzahl von Formwerkzeugen, welche Formwerkzeuge dazu ausgebildet sind, zum Formen des Formkörpers aus mindestens einer Materialbahn mit einer Fördereinrichtung für die Materialbahn zusammenzuwirken, in welcher Formstation die Formwerkzeuge zumindest während einer zum Formen des Formkörpers benötigten Einwirkzeit in einem Bearbeitungsbereich synchron zusammen mit der Materialbahn bewegbar und dabei in wenigstens einer ersten geschlossenen Werkzeugführungsstruktur geführt sind, wobei die Werkzeugführungsstruktur zumindest in dem Bearbeitungsbereich einen geraden Abschnitt aufweist und dort im Wesentlichen parallel zur Materialbahn verläuft, dadurch gekennzeichnet, dass zum Bewegen der Formwerkzeuge wenigstens eine Antriebseinrichtung vorgesehen ist, welche dazu ausgebildet ist, auf wenigstens eines der Formwerkzeuge einzuwirken, wobei die Formwerkzeuge in der Werkzeugführungsstruktur im Wesentlichen lückenlos angeordnet sind und schiebend aufeinander einwirken.

Eine grundlegende Eigenschaft der vorliegenden Erfindung besteht somit darin, dass an Stelle des Reibradantriebs eine Art Schubantrieb für die Formwerkzeuge vorgesehen ist. Die vorhandene Antriebseinrichtung, bei der es sich vorteilhafter Weise um eine synchron zu der bewegten Materialbahn rotierende Schneckenwelle handelt, wie sie bereits aus der DE 10 2007 030 008 bekannt ist, durch die die Formwerkzeuge entsprechend mitgeführt werden, wirkt somit lediglich auf ein einzelnes Formwerkzeug oder eine relativ kleine Anzahl von Formwerkzeugen im Bearbeitungsbereich (direkt) ein, während die restlichen Formwerkzeuge durch die Schubwirkung der angetriebenen Formwerkzeuge (indirekt) entlang der geschlossenen Werkzeugführungsstruktur bewegt werden. Auf diese Weise kann auf den Reibradantrieb vollständig verzichtet werden, wodurch die oben angesprochenen Probleme vermieden werden.

Vorteilhafter Weise kann somit generell auf das Vorsehen zusätzlicher Antriebe, wie sie im Falle der DE 10 2007 030 008 in Form der Reibräder erforderlich waren, verzichtet werden, wenn die zum synchronen Bewegen von Materialbahn und Formwerkzeugen ohnehin regelmäßig vorzusehende Antriebseinrichtung auch zur Realisierung des Schubantriebs verwendet wird, was einen entsprechenden Kostenvorteil bedingt.

Dabei erfolgt die Bewegung von Formwerkzeug und der Materialbahn während der Einwirkzeit weiterhin synchron, so dass in Förderrichtung der Materialbahn keine Relativbewegung zwischen Formwerkzeug und Materialbahn auftritt.

Der Begriff "Formwerkzeug", wie er im Rahmen dieser Offenbarung verwendet wird, umfasst neben dem eigentlichen Werkzeug, welches auf die Materialbahn einwirkt, auch eine Vehikelanordnung (beispielsweise in Form eines Laufwagens), mit welcher das eigentliche Werkzeug über eine Werkzeughalterung oder Werkzeugaufnahme verbunden und entsprechend entlang der Werkzeugführungsstruktur geführt beweglich ist, beispielsweise durch Rollen, Gleiten, Schweben oder dergleichen. Wenn im Rahmen der Ausführungsbeispiele nur auf ein Rollen der Formwerkzeuge entlang der Werkzeugführungsstruktur näher eingegangen wird, bedeutet dies keine Beschränkung der vorliegenden Erfindung.

Die Formstation kann im Bereich der Werkzeugführungsstruktur neben geraden Abschnitten auch gekrümmte Abschnitte aufweisen, wobei insbesondere die gekrümmten Abschnitte halbkreisförmig ausgebildet und die geraden Abschnitte zwischen den halbkreisförmigen Abschnitten angeordnet sind. Die Werkzeugführungsstruktur ist jedoch nicht auf derartige Formgebungen insbesondere der gekrümmten Abschnitte beschränkt, sondern kann grundsätzlich jede (geschlossene) Form annehmen. Es können auch mehrere Werkzeugführungsstrukturen vorhanden sein.

Die konkrete Anzahl von Formwerkzeugen, die bei einer erfindungsgemäßen Formstation vorhanden sind, um die Werkzeugführungsstruktur zwecks Realisierung des Schubantriebs im Wesentlichen lückenlos zu füllen (Vollbesetzung), und die nach einem Umlauf entlang der Werkzeugführungsstruktur wieder zum erneuten Bearbeiten der Materialbahn zur Verfügung stehen, hängt unter anderem von der Länge der Werkzeugführungsstruktur ab Außerdem spielt das Format (oder die Größe) der Formkörper bzw. die entsprechende Formatlänge der Formwerkzeuge eine entscheidende Rolle.

In der Praxis ist bei Format- bzw. Werkzeugwechsel eine Anpassung der Länge der Werkzeugführungsstruktur entweder unmöglich oder mit erheblichem Aufwand verbunden und deshalb nicht wünschenswert, während sich die Antriebseinheit, insbesondere bei Ausbildung als Schneckenwelle über die Steigung, relativ leicht an unterschiedliche Formale anpassen lässt oder gar keine solche Anpassung benötigt. Dabei tritt jedoch die Problematik auf, dass bei gegebener Länge der Werkseugführungsstruktur und gleichfalls gegebener Formwerkzeug-abmessung (Formatlänge) in der Regel eine Besetzungslücke verbleiben wird, die kein zusätzliches Formwerkzeug mehr aufnehmen kann.

In diesem Zusammenhang kann zunächst zwecks Realisierung eines definierten Abstands zwischen in der Werkzeugführungsstruktur aufeinander folgenden Formwerkzeugen bei einer Weiterbildung der erfindungsgemäßen Formstation vorgesehen sein, dass wenigstens eines der Formwerkzeuge eine erste Distanzhalteeinrichtung aufweist, welche einen ersten minimalen Abstand zu einem benachbarten Formwerkzeug in der Werkzeugführungsstruktur definiert. Bei dieser ersten Distanzhalteeinrichtung kann es sich um einen an dem Formwerkzeug angeordneten Vorsprung handelt, der mechanisch mit einem entsprechenden Bereich (Anlagebereich) des benachbarten Formwerkzeugs zusammenwirkt. Das Zusammenwirken ist jedoch nicht auf rein mechanisches Zusammenwirken beschränkt, insbesondere auch (elektro-)magnetisches Zusammenwirken kommt in Betracht, wobei sich über gleichnamige Pole bzw. Polung eine berührungsfreie Abstoßung und damit die erwünschte Schubwirkung realisieren lässt

Vorzugsweise kann die erste Distanzhalteeinrichtung dazu ausgebildet sein, bei im Wesentlichen geradliniger Führung des Formwerkzeugs in der Werkzeugführungsstruktur mit einem entsprechenden ersten Anlagebereich an dem benachbarten Formwerkzeug zusammenzuwirken. Zu diesem Zweck kann sie entlang einer Längsachse des Formwerkzeugs bzw. der zugehörigen Vehikelanordnung orientiert sein.

Der durch die erste Distanzhalteeinrichtung vorgegebene erste Abstand solite so ausgelegt rein, dass sichergestellt ist, dass im Eingangs- bzw. Einlaufbereich des geraden Bearbeitungsbereichs die Formwerkzeuge einen geeigneten Abstand voneinander aufweisen, um problemios von den Antriebseinheit aufgenommen und weitertransportiert zu werden. Dabei ist der erste Abstand bzw. ein daraus resultierender Abstand zwischen Formwerkzeug-Strukturen, die zum Zusammenwirken mit der Antriebseinilelt vorgesehen sind, beispielsweise in Form von (Transport-)Rollen, an die Antriebseinheit angepasst, im Falle der erwähnten Schneckenwelle also beispielsweise an die Steigung der Schneckenwelle, wobei der erste Abstand einem Abstand benachbarter Formwerkzeuge während des Antreibens selbst - also der Formatlänge - bis auf ein mögliches geringes Mindermaß im Wesentlichen entspricht. Somit ist gewährleistet, dass die Antriebseinrichtung die einlaufenden Formwerkzeuge sicher aufnimmt und weitertransportiert. Der erste Abstand ist somit in Anpassung an die verwendete Antriebseinrichtung im Wesentlichen festgelegt.

Um nun insbesondere die weiter oben erwähnte Besetzungslücke auszugleichen, damit der Schubantrieb unabhängig von der Formatlänge bei vorgegebener Länge der Werkzeugführungsstruktur sicher funktioniert, kann bei einer anderen Weiterbildung der erfindungsgemäßen Formstation vorgesehen sein, dass das wenigstens eine Formwerkzeug noch eine zweite Distanzhaiteeinrichtung aufweist, welche einen zweiten Abstand zu einem benachbarten Formwerkzeug in der Werkzeugführungsstruktur definiert.

Die zweite Distanzhalteeinrichtung kann in diesem Zusammenhang weiterhin dazu ausgebildet sein, bei krummliniger Führung des Formwerkzeugs in der Werkzeugführungssiruktur mit einem entsprechenden zweiten Anlagebereich an dem benachbarten Formwerkzeug zusammenzuwirken. Mit anderen Worten: die zweite Distanzhalteeinrichtung bzw. der zweite Abstand zwischen benachbaren Formwerkzeugen kommt nur bei Kurvenführung (nicht geradliniger Führung) innerhalb der Werkzeugführungsstruktur zum Tragen und behindert somit nicht das Einlaufen in den geraden Bearbeitungsbereich bzw. das Zusammenwirken mit der Antriebseinrichtung, wie weiter oben beschrieben. Wenn dann weiterhin der zweite Abstand betragsmäßig von dem ersten Abstand abweicht und insbesondere größer ist als der erste Abstand, lässt sich die Besetzungslücke schließen.

Um hierbei eine größere Flexibilität zu erhaben und/oder den Betrieb des erfindungsgemäßen Formstation fein abzustimmen, kann bei einer anderen Weiterbildung derselben vorgesehen sein, dass der erste Abstand und/oder der zweite Abstand einstellbar ist, vorzugsweise stufenlos, beispielweise durch geeignete Schraubmittel, Gewindemittel, Klemmmittel, Rastmittel und/oder durch Einsetzen von Abstand-/Distanzstücken oder dergleichen.

Auf diese Weise kann erreicht werden, dass die im Wesentlichen lückenlose Anordnung der Formwerkzeuge in der Werkzeugführungsstruktur auch bei Änderung einer Formatlänge der Formkörper und/oder bei Änderung einer Formwerkzeug-Abmessung ohne größeren Konstruktions- oder Montageaufwand realisierbar ist.

Um Verschleiß und Abnutzungserscheinungen beim Schieben der Formwerkzeuge vorzubeugen, kann bei der erfindungsgemäßen Formstation weiterhin vorgesehen sein, dass die erste Distanzhalteeinrichtung und/oder die zweite Distanzhalteeinrichtung und/oder eine eventuell vorgesehene zusätzliche dritte Distanzhalterung elastisch-nachgiebig ausgebildet ist bzw. sind, vorzugsweise durch Vorsehen von Federmitteln. Puffermitteln, Dämpfungsmitteln oder dergleichen.

Insbesondere damit es beim Schieben der Formwerkzeuge auch bei Kurvenfahrt oder im Übergang zwischen gekrümmten und geradlinigen Abschnitten der Werkzeugführungsstruktur nicht zu Verkantungen und/oder übermaßiger Abnutzung kommt, ist im Zuge einer anderen Weiterbildung der erfindungsgemäßen Formstation vorgesehen, dass die erste Distanzhalteeinrichtung und/oder die zweite Distanzhalteeinrichtung und/oder eine zusätzliche dritte Distanzhalterung an ihrem jeweiligen freien Ende, welches für das Zusammenwirken mit einem benachbarten Formwerkzeug vorgesehen ist, eine Abrundung aufweist.

Vorzugsweise befinden sich zumindest einige der vorstehend genannten Distanzhalteeinrichtungen nicht an dem Werkzeug selbst, sondern an der weiter oben erwähnten Vehikelanordnung (Laufwagen), mit welcher das eigentliche Werkzeug über eine Werkzeughalterung oder Werkzeugaufnahme verbunden und entsprechend entlang der Werkzeugführurtgsstruktur geführt ist.

Die eigentlichen Werkzeuge zum Einwirken auf die zwecks Herstellung der Formkörper, welche Teil der Formwerkzeuge sind, wurden in der DE 10 2007 030 008 detailliert beschrieben, worauf an dieser Stelle nochmals explizit Bezug genommen wird. Die Werkzeuge selbst sind nicht Gegenstand der vorliegenden Erfindung. Sie können obere und untere Formeinheiten aufweisen, die zum Formen des Formkörpers gegeneinander bewegbar sind. Vorteilhafterweise erfolgt diese Bewegung senkrecht zu einem Verkauf der Materialbahn, die entsprechend zwischen den genannten Formeinheiten angeordnet ist.

Das Gegeneinanderbewegen der oberen und unteren Formeinheiten kann durch Zwangsführung mittels einer zweiten Werkzeugführungsstruktur erfolgen, wie in der DE 10 2007 030 008 beschrieben. Diese ist zu diesem Zweck derart ausgestaltet, dass ihr Abstand von der Materialbahn innerhalb des Bearbeitungsbereichs zunächst ab- und anschließend wieder zunimmt.

Weiterhin kann vorgesehen sein, dass die Formwerkzeuge als kombinierte Stand- und Formwerkzeuge zum Stanzen der materialbahn und zum Formen des Formkörpers aus dem ausgestanzten Formkörpermaterial ausgebildet sind. Zum Einwirken auf die Materialbahn bzw. das Formkörpermaterial weist eine der beiden Formeinheiten ein Patrizenteil und die andere Formeinheit ein im Wesentlichen dazu komplementär ausgebildetes Matrizenteil auf. Um das Formkörpermaterial nicht zu beschädigen, insbesondere wenn dieses ein Absorption steigerndes Granulat enthält, kann vorgesehen sein, dass entweder das Patrizenteil oder das Matrizenteil eine Beschichtung aufweist, die relativ zu dem Material des jeweils anderen Teils welcher ist.

Insbesondere wenn mehrschichtiges Material mit einer absorbierenden Schicht und diese einschließenden Fluid durchlässigen bzw, Fluid undurchlässigen Schichten verarbeitet wird, kann vorgesehen sein, dass das Formwerkzeug eine Heizeinrichtung zum thermischen Einwirken (Laminieren) auf das Formkörpermaterial aufweist.

Insbesondere zum Fixieren des Formkörpermaterials kann das Formwerkzeug Außerdem mit Vakuum beaufschlagt werden.

Damit es nicht zum einer Beschädigung des Formkörpermäterials kommt, ist vielfach erforderlich, die Heizwirkung der Heizeinrichtung genau zu überwachen und entsprechend zu regeln. Zu diesem Zweck ist Vorteilhafterweise eine entsprechende Schalt- und Vorgesehen. Diese dient einerseits - wie gesagt - zum Überwachen und Regeln der Heizwirkung, andererseits jedoch auch grundsätzlich zur Energieversorgung der umlaufenden Formwerkzeuge.

Um der besonderen Problematik zu begegnen, die sich dadurch ergibt, dass die Formwerkzeuge eine umlaufende Bewegung ausführen, kann zusätzlich vorgesehen sein, dass die Schalt- und Steuereinrichtung bzw. eine mit dieser zusammenwirkende Anschlusseinrichtung zum kabelgebuncienen Verbinden der Schalt- und Steuereinrichtung mit den Formwerkzeugen zusammen mit letzteren bewegt ist, insbesondere in Form einer Drehbewegung. Die primäre Energieversorgung erfolgt dann beispielsweise unter Verwendung von Schleifringkörpern oder dergleichen.

Bei einer anderen Ausgestaltung weist die drehbare Schalt- und Steuereinrichtung einen eigenen Antrieb auf, so dass die Drehbewegung unabhängig von einem Umlauf der Formwerkzeuge erfolgen kann, um einer Verwirrung der Kabelverbindungen zu den einzelnen Formwerkzeugen vorbeugen bzw. begegnen zu können.

Bezüglich ihrer Verwendung eignet sich die erfindungsgemäße Formstation - auch im Zuge ihrer verschiedenen Weiterbildungen - insbesondere zur Verarbeitung eines zusammengesetzten Materials mit wenigstens einer saugfähigen, insbesondere Granulat angereicherten Schicht sowie weiteren Fluid dichten oder Fluid durchlässigen Schichten.

Weitere Eigenschaften und Vorteile der vorliegenden Erfindung ergeben sich aus der nachfolgenden Beschreibung von Ausführungsbeispielen anhand der Zeichnung. Es zeigt:
- Figur 1: eine Draufsicht auf eine erfindungsgemäße Formstation;
- Figur 2: eine schematische Ansicht zweier benachbarter FormwerkzeugLaufwagen für eine erfindungsgemäße Formstation bei geradliniger Bewegung entlang der Werkzeugführungsstruktur,
- Figur 3: die Formwerkzeug-Laufwagen aus Figur 2 bei geradliniger Bewegung in direkter Wechselwirkung mit der Antriebseinrichtung (Schneckenwelle);
- Figur 4: die Formwerkzeug-Laufwagen aus Figur 2 und 3 bei krummliniger Bewegung entlang der Werkzeugführungsstruktur ohne zweite Distanzhalteeinrichtung;
- Figur 5: die Formwerkzeug-Laufwagen aus Figur 2 und 3 bei krummliniger Bewegung entlang der Werkzeugführungsstruktur mit zweiter Distanzhalteeinrichtung; und
- Figur 6: eine detaillierte perspektivische Ansicht eines Formwerkzeug-Laufwagens für eine erfindungsgemäße Formstation.

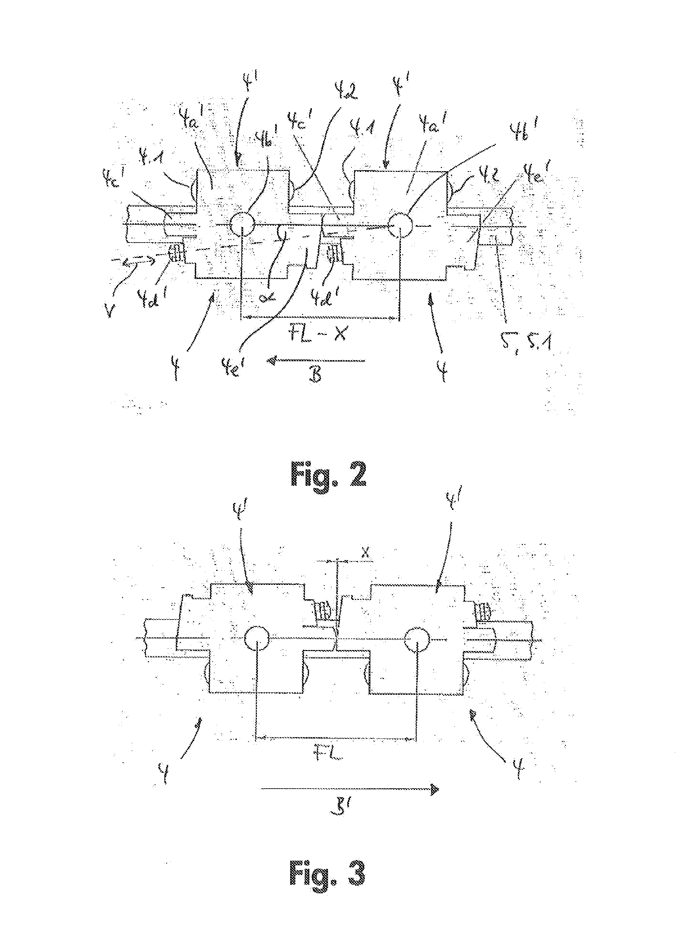

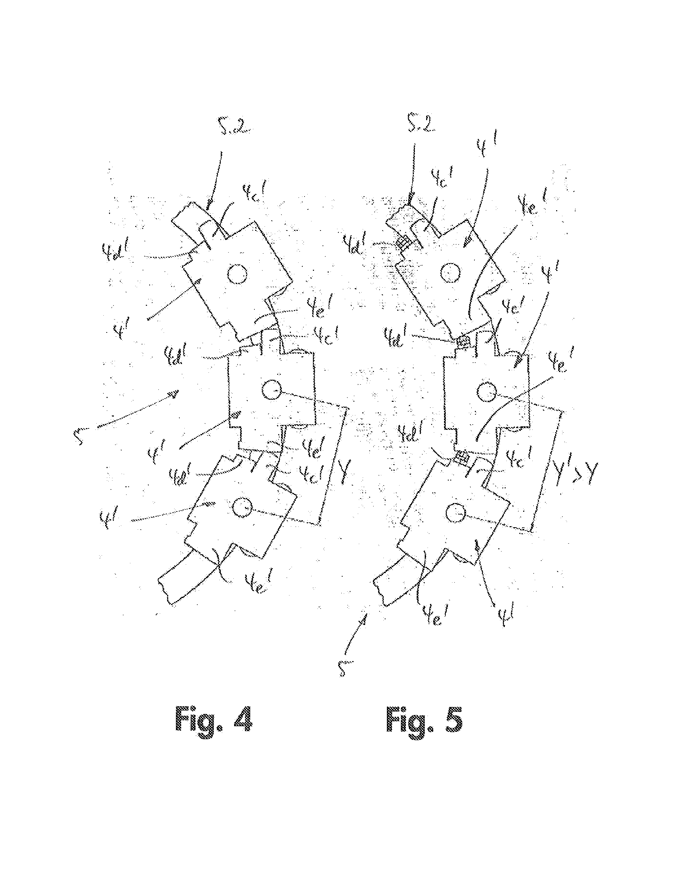

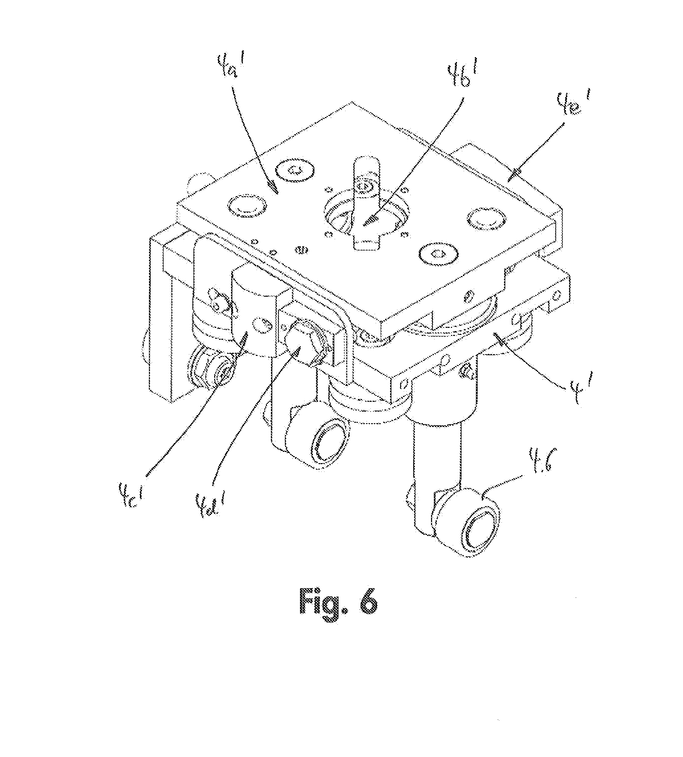

Die nachfolgend beschriebenen exemplarischen Ausgestaltungen einer erfindungsgemaßen Formstation können zusätzlich zu den jeweils explizit erwähnten Merkmalen auch noch solche Merkmale aufweisen, wie sie anhand der Figuren 2 bis 7 der DE 10 2007 030 008 beschrieben sind Dies betrifft insbesondere die Schalt- und Steuereinrichtung (Drehanordnung) gemäß den Figuren 6 und 7 der DE 10 2007 030 008, die oberen/unteren Formeinheiten der verwendeten Formwerkzeuge gemäß den Figuren 4 und 5 der DE 10 2007 030 008 bzw. die vierwendeten Formwerkzeuge selbst sowie die Zwangsführung durch die Werkzeugführungsstrukturen im Bearbeitungsbereich der Formstation (vgl. insbesondere Figur 2 aus DE 10 2007 030 008, dort Bezugszeichen "9") zur Realisierung des eigentlichen Formgebungsprozesses. Auf alle diese Merkmale wird hiermit nochmals ausdrücklich Bezug genommen, da sie auch beim Gegenstand der vorliegenden Erfindung verwirklicht sein können.

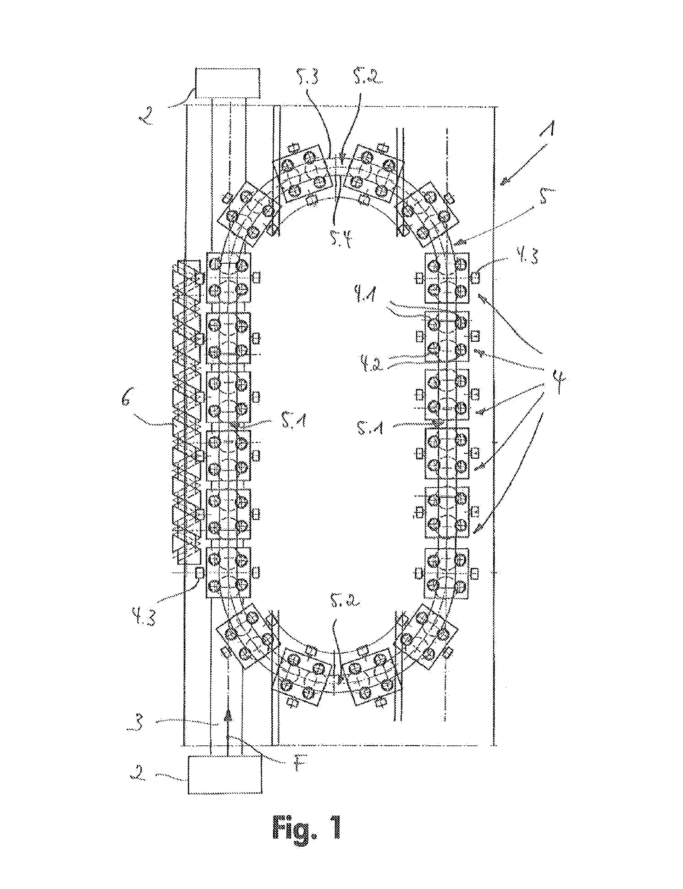

Nachfolgend wird anhand der beigefügten Figuren 1 bis 6 vornehmlich auf die im Rahmen der vorliegenden Erfindung hinzugekommenen Neuerungen näher eingegangen:

Figur 1 zeigt schematisch eine Draufsicht auf eine erfindungsgemäße Formstation 1 zum Herstellen von Formkörpern, insbesondere Stilleinlagen oder dergleichen. Die Formstation 1 weist zunächst eine nur andeutungsweise dargestellt Fördereinrichtung 2 für eine Materialbahn 3 auf, die entlang einer Förderrichtung F durch die Formstation 1 gefördert wird. Bei der Materialbahn 3 handelt es sich vorzugsweise um eine Bahn aus einem zusammengesetzten Material, das beispielsweise eine saugfähige Schicht aus Zellulose oder dergleichen (gegebenenfalls mit einem die Absorption steigernden Granulat angereichert) sowie weitere Schichten aus Fluid durchlässigem und Fluid undurchlässigem Material aufweist. Weiterhin weist die Formstation eine Mehrzahl von Formwerkzeugen 4 auf, die vorliegend allesamt identisch ausgebildet sind, wobei die vorliegende Erfindung jedoch nicht auf eine derartige Ausführungsform beschränkt ist.

Auf die Ausgestaltung der Formwerkzeuge 4 selbst bzw. der darin enthaltenen Formeinheiten wurde in der DE 10 2007 030 008 bereits detailliert eingegangen (vgl. die dortigen Figuren 2 bis 5 mit Beschreibung). Sie dienen zum Verprägen bzw. Versiegeln des Rands eines Formkörpermaterials aus der Materialbahn 3 sowie zum Formen der herzustellenden Formkörper aus dem Formkörpermaterial und wirken zu diesem Zweck mit der Fördereinrichtung 2 zusammen, worauf weiter unten ebenfalls noch genauer eingegangen wird. Grundsätzlich ist auch möglich, die Formwerkzeuge 4 als kombinierte Stanz- und Formwerkzeuge ausdurch die zunächst das Formkörpermaterial aus der Materialbahn 3 ausgestanzt und verprägt und anschießend geformt wird,

Die Formstation 1 weist weiterhin eine geschlossenen Werkzeugführungsstruktur 5 auf, die sich nach Art einer Stadion-Laufbahn aus zwei geraden Abschnitten 5.1 zusammensetzt, die durch halbkreisförmig gekrümmte Abschnitte 5.2 verbunden sind. Die Formwerkzeuge 4 sind entlang der geschlossenen Werkzeugführungsstruktur 5 zwangsgeführt und weisen zu diesem Zweck jeweils zwei Rollenpaare 4.1, 4.2 auf, deren einzelne Rollen jeweils derart voneinander beanstandet sind, dass sie seitlich beiderseits mit der Werkzeugführungsstruktur 5 an deren Außen- und innenkante 5.3 bzw. 5.4 zusammenwirken. Auf diese Weise können die Formwerkzeuge 4 entlang der Werkzeugführungsstruktur 5 umlaufen, worauf bereits in der DE 10 2007 030 008 detailliert eingegangen wurde.

Zum Antreiben der Formwerkzeuge 4 bei ihrem Umlauf entlang der geschlossenen Werkzeugführungsstruktur 5 ist zunächst eine Antriebseinrichtung in Form einer Schneckenwelle 6 vorgesehen, die parallel zur Förderrichtung F der Materialbahn 3 angeordnet ist und die bei dem gezeigten Ausführungsbeispiel synchron zur Bewegung der Materialbahn 3 rotiert. Die Formwerkzeuge 4 weisen äußere Kurvenrollen 4,3 auf, die im Bereich der Schneckenwelle 6 mit deren Gewindestruktur in Eingriff treten, so dass die Formwerkzeuge 4 im Bereich der Schneckenwelle 6 synchron mit der Förderbewegung der Materialbahn 3 bewegt sind. Mit anderen Worten: Im Bereich der Schneckenwelle 6 findet auch bei kontinuierlich geförderter Materialbahn 3 keine Relativbewegung zwischen den Formwerkzeugen 4 und der Materialbahn 2 bezogen auf die Förderrichtung F der Materialbahn 3 statt. Während dieser Zeit kann somit erfindungsgemäß ein Einwirken der Formwerkzeuge 4 auf die Materialbahn 3 erfolgen, auch wenn diese kontinuierlich gefördert wird. Die Länge der Schneckenwelle 6 bzw. die Länge der entsprechenden Graden 5, 1 der geschlossenen Werkzeugführungsstruktur 5 sowie die Fördergeschwindigkeit der Materialbahn 3 und entsprechend die Rotation der Schneckenwelle 6 sind so bemessen und aufeinander abgestimmt, dass die resultierende Einwirkzeit zum Verprägen des Formkörpermaterials aus der Materialbahn 3 und zum anschließenden Formen des Formkörpers aus dem Formkörpermaterial ausreicht.

Der Bereich der Formstation 1 entlang der Schneckenwelle 6, in dem - wie vorstehend beschrieben - das Verprägen und Formen des Formkörpers stattfindet, wird vorliegend auch als Bearbeitungsbereich bezeichnet. Nach Durchlaufen dieses Bearbeitungsbereichs werden die Formwerkzeuge 4 entlang der geschlossenen Werkzeugführungsstruktur 5 wieder zum Beginn des Bearbeitungbereichs geführt wo sie für ein erneutes Einwirken auf die Materialbahn 3 zur Verfügung stehen. Zu diesem Zweck weist die Formstation 1 - anders als die aus der DE 10 2007 030 008 bekannte Formstation - keine weiteren aktiven Antriebsstrukturen für die Formwerkzeuge 4 in Form von Reibrädern oder dergleichen auf, sondern realisiert den Vortrieb der Formwerkzeuge 4 entlang der Werkzeugführungsstruktur 5 mittels eines Schubantriebs, bei dem jedes Formwerkzeug 4 schiebend auf das in Bewegungsrichtung (gemäß Figur im Uhrzeigersinn) vorangehende Formwerkzeug 4 einwirkt und die Antriebskraft letztendlich allein durch die Schneckenwelle 6 vermittelt wird.

Zu diesem Zweck ist es erforderlich, dass die Formwerkzeuge 4 die Werkzeugführungsstruktur 5 im Wesentlichen lückenlos auffüllen, wie dies in Figur 1 andeutungsweise dargestellt ist. Die schiebend aufeinander einwirkenden Strukturen zwischen den einzelnen Formwerkzeugen 4 sind in Figur 1 lediglich aus Gründen der Übersichtlichkeit nicht explizit dargestellt und werden nachfolgend anhand der Figuren 2 bis 6 noch genauer erläutert. Wie dem Betrachter auffälit, ist der Abstand zwischen den einzelnen Formwerkzeugen 4 in den geraden Bereichen 5, 1 der Werkzeugführungsstruktur 5 von demjenigen Abstand verschieden, welchen die Formwerkzeuge 4 in den gekrümmten Bereich in 5,2 der Werkzeugführungsstruktur 5 einnehmen. Auch hierauf wird weiter unten noch genauer eingegangen.

Selbstverständlich ist die Erfindung nicht auf das vorstehend beschriebene Ausführungsbeispiel beschränkt, insbesondere kann die Umlaufrichtung auch entgegen dem Uhrzeigersinn gewählt sein. Außerdem können mehrere Werkzeugfuhrungsstrukturen zum Transportieren der Formwerkzeuge vorgesehen sein, beispielsweise zwei gegensinnig zu durchlaufene Führungsstrukturen, die im Wesentlichen nach Art der Ziffer "8" angeordnet sein können. Des Weiteren ließe sich grundsätzlich auch in dem geraden Bereich 5,1 der Werkzeugführungsstruktur 5 noch eine weitere Materialbahn mit zugehöriger Fördereinrichtung anordnen, um einen effektiv vergrößerten Bearbeitungsbereich zu schiffen,

Anders als beim Gegenstand der DE 10 2007 030 008 ist bei der Formstation 1 gemaß Figur 1 aufgrund der lückenlosen Auffüllung der Werkzeugführungsstruktur 5 mit Formwerkzeugen 4 ein Austauschen der Formwerkzeuge 4 nur bei insgesamt gleich bleibender Werkzeuganzahl bzw. konstanter Werkzeugabmessung in Bewegungsrichtung (vorliegend auch als Formatlänge bezeichnet) ohne Weiteres möglich. Auf diese besondere Problematik wird weiter unten noch genauer eingegangen.

Wie in der DE 10 2007 030 008 beschrieben (vgl. dort insbesondere Figur 2), sind die Formwerkzeuge 4 zweiteilig mit einer oberen Formeinheit und einer unteren Formeinheit ausgebildet, von denen eine oberhalb und die andere unterhalb einer Ebene der Materialbahn 3 angeordnet ist. Für jede dieser Formeinheiten ist jeweils eine eigene Werkzeugführungsstruktur 5 vorgesehen, was der Darstellung in der vorliegenden Figur 1 nicht zu entnehmen ist. Allgemein ist die Formstation 1 hinsichtlich der Antriebseinrichtungen für die Formwerkzeuge 4 (Fuhrungsstruktur 5, Schneckenwelle 6) angesichts der zweiteiligen Ausführung der Formwerkzeuge 4 bezüglich der Materialbahnebene symmetrisch ausgebildet, worauf bereits in der Voranmeldung detailliert eingegangen wurde, so dass sich weitere Ausführungen diesbezüglich erübrigen. Dies betrifft insbesondere auch die an den Formwerkzeugen vorgesehenen Rollenanordnungen und deren Wechselwirkung mit der betreffenden Werkzeugführungsstruktur 5.

Aufgrund der umlaufenden Formwerkzeuge 4 stellt deren versorgungs- und steuerungstechnische Anbindung, beispielsweise zur Stromversorgung und zur Überwachung der Heizwirkung, vorliegend eine besondere Problematik dar. Die DE 10 2007 030 008 zeigt im Rahmen der dortigen Figuren 6 und 7 Ansichten eines Ausführungsbeispiels für eine Schalt-, Steuer- und Versorgungseinrichtung, die auch bei der erfindungsgemäßen Formstation zum Einsatz kommen kann. Auf die entsprechende Offenbarung wird verwiesen.

Figur 2 zeigt schematisch zwei aufeinander folgende Formwerkzeuge 4, welche in Bewegungsrichtung B entlang bzw. auf der Werkzeugführungsstruktur 5 geführt bzw. bewegt sind. Dargestellt sind bei Bezugszeichen 4' lediglich so genannte Vehikelanordnungen in Form von Laufwagen für die Formwerkzeuge 4, auf welchen Laufwagen 4' anschließend die eigentlichen Werkzeuge (obere/untere Formeinheiten) montierbar sind, worauf bereits weiter oben und auch in der DE 10 2007 030 008 (dort zur Figur 2) hingewiesen wurde.

Bei dem dargestellten Teilstück der Werkzeugführungsstruktur 5 handelt es sich in Figur 2 um einen geraden Abschnitt 5.1 außerhalb des Bearbeitungsbereichs (vgl. Figur 1).

Die Laufwagen 4' weisen in der Draufsicht einen rechteckigen, angenährt quadratischen plattenförmigen Grundkörper 4a' auf, welcher Grundkörper als Werkzeugaufnahme dient und zu diesem Zweck weiterhin eine zentrale Befestigungsstruktur 4b' in Form einer in etwa kreiszylindrischen Ausnehmung aufweist, ohne dass die vorliegende Erfindung jedoch auf eine derartige Ausgestaltung beschränkt wäre. An dem Grundkörper sind weiterhin noch Rollen 4.1, 4 2 angeordnet, welche zwecks Führung der Laufwagen 4' mit der Werkzeugführungsstruktur 5 in Wechselwirkung treten, wie weiter oben anhand von Figur 1 bereits beschrieben und grundsätzlich auch aus der DE 10 2007 030 008 bekannt.

Des Weiteren sind an dem Grundkörper 4a' eine Reihe von Distanzhalteeinrichtungen vorgesehen, welche im Rahmen der vorliegenden Erfindung von besonderer Bedeutung sind und auf weiche nachfolgend genauer eingegangen werden soll:
Eine erste Distanzhalteeinrichtung 4c' ist bei Geradeausfahrt in Vorwärtsrichtung der Laufwagen 4' angeordnet. Sie ist nach Art eines quaderförmigen Vorsprungs mit abgerundeter Vorderkante ausgebildet, wie aus Figur 2 ersichtlich.
Quer zur Fahrtrichtung weist die Distanzhalteeinrichtung 4c' bezogen auf den gesamten Laufwagen 4' eine relativ geringe Abmessung auf und ist damit im Wesentlichen auf den zentralen Bereich des Grundkörpers 4a' begrenzt.

Bei Bezugszeichen 4d' ist jeweils eine zweite Distanzhalteeinrichtung vorgesehen, welche gemäß der Ausgestaltung in Figur 2 seitlich neben der ersten Distanzhalteeinrichtung 4c' auf derselben Seite des Grundkörpers 4a' angeordnet ist. Die zweite Distanzhalteenrichtung 4d' ist bezogen auf die Richtung B bzw. die Erstreckung des geraden Werkzeugsführungsstruktur-Abschnitts 5.1 schräg unter einem Winkel a orientiert - und zwar nach innen bezogen auf die Werkzeusführungsstruktur 5, was insbesondere unten anhand von Figur 4 und Figur 5 noch deutlicher werden wird. In Fahrtrichtung B weist die zweite Distanzhalteeinrichtung 4d' ein Mindermaß gegenüber der ersten Distanzhaltereinrichtung 4c' auf. Dies führt dazu, dass bei Geradeausfahrt der Laufwagen 4', wie in Figur 2 dargestellt, eine Berührung zwischen aufeinander folgenden Laufwagen 4' nur im Bereich der ersten Distanzhalteeinrichtung 4c' stattfindet, wie im mittleren Bereich der Figur 2 erkennbar ist. Die angesprochene Wechselwirkung der ersten Distanzhalteeinrichtung 4c des nachfolgenden Laufwagens 4' (rechts in Figur 2) mit dem vorhergehenden Laufwagen 4' (links in Figur 2) erfolgt im Bereich einer dritten Distanzhalteeinrichtung 4e', welche bezogen auf die Bewegungsrichtung B auf der Rückseite des Grundkörpers 4a' angeordnet ist und dabei als Anlagebereich für die erste Distanzhalteeinrichtung 4c' fungiert. Dabei entspricht die Breite der dritten Distanzhalteeinrichtung 4e', das heißt ihre Erstreckung quer zur Bewegungsrichtung B einer Abmessung, welche größer ist als die Abmessung desjenigen Bereichs, welcher durch die erste Distanzhalteeinrichtung 4c' und die zweite Distanzhalteeinrichtung 4d' aufgespannt wird. Des Westeren ist die dritte Distanzhalteeinrichtung 4e' an ihrem freien, von dem Grundkörper 4a' abgewandten Ende bezogen auf die Bewegungsrichtung B abgeschrägt ausgebildet. Die Abschrägung ist derart gewählt, dass sie in etwa spiegelbildlich zu einer (gedachten) Verbindungslinie zwischen den freien Enden der ersten und zweiten Distanzhalteeinrichtungen 4c', 4d' verläuft, wie ebenfalls im mittleren Bereich der Figur 2 erkennbar ist.

Während die dritte Distanzhalteeinrichtung 4e' zwar abgeschrägt bezüglich der Laufwagen-Längsachse aber ansonsten geradlinig ausgebildet ist, weist die zweite Distanzhalteeinrichtung 4d' an ihrem freien Ende ebenso wie die erste Distanzhalteeinrichtung 4c' eine Abrundung auf, wie der Figur 2 zu entnehmen ist.

Der Abstand zwischen den Werkzeugbefestigungsstrukturen 4b' unmittelbar aufeinander folgender Formwerkzeuge 4, deren Laufwagen 4' sich also gemäß Figur 2 im Bereich der ersten Distanzhalteeinrichtung 4c' und der dritten Distanzhalteeinrichtung 4e' berühren, beträgt FL - X, wobei FL die bereits erwähnte Formatlänge und X eine (betragsmäßig relativ kleine) Abweichung von der Formaltänge FL bezeichnet. Mit anderen Worten: In den geraden Bereichen 5.1 der Werkzeugführungsstruktur 5 werden die Formwerkzeuge 4 mit einem effektiven Abstand FL - X bewegt, welcher nur wenig von der gewähren Formatlänge FL abweicht. Dies betrifft jedoch nur diejenigen geraden Abschnitte 5.1 der Werkzeugführungsstruktur 5, in denen die Formwerkzeuge 4 nicht durch die Schneckenwelle 6 direkt angetrieben sind. Auf letzteren Fall wird weiter unten anhand von Figur 3 noch genauer eingegangen.

Gemäß der Darstellung in Figur 2 schiebt also jedes Formwerkzeug 4 mit seiner vorderen, ersten Distanzhalteeinrichtung 4c' immer das vorausfahrende Formwerkzeug 4 im Bereich von dessen dritter Distanzhalteeinrichtung 4e' an, so dass über die lückenlose Füllung der Werkzeugführungsstruktur 5 mit Formwerkzeugen 4 (vgl. Figur 1) ein Schubantrieb für die Formwerkzeuge 4 realisiert ist.

In Figur 3 ist der Sachverhalt gezeigt, wie er sich darstellt, wenn die Formwerkzeuge 4 bzw. die zugehörigen Laufwagen 4' sich im Bereich der Schneckenwelle 6 (vgl. Figur 1) befinden und von dieser direkt angetrieben bzw. synchron mit der Materialbahn 3 bewegt werden. Der effektive Abstand zwischen den FormWerkzeugen 4 entspricht dann genau der Formatlänge FL, ist also um den Betrag X größer als bei der Darstellung gemäß Figur 2. Die Schneckenwelle und die damit zusammenwirkenden Kurvenrollen 4.3 (vgl. Figur 1) sind in Figur 3 nicht explizit dargestellt. Die Bewegung erfolgt in Richtung des Pfeils B' von links nach rechts. Gemäß der Darstellung in Figur 3 kommt es im Bereich der Schneckenwelle zu keiner direkten Berührung zwischen benachbarten Formwerkzeugen 4 bzw. Laufwagen 4'. Der Weitertransport erfolgt in diesem Bereich nicht durch Schubwirkung zwischen den einzelnen Formwerkzeugen, sondern durch direkten Antrieb mittels der Schneckenwelle, wie bereits mehrfach erwähnt. Die Schneckenwelle selbst ist hinsichtlich ihrer Ausgestaltung (Steigung) auf die Formatlänge FL abgestimmt. Durch die betragsmäßig nur kleine Abweichung X (vgl. Figur 2) bezüglich der Formatlänge und dem entsprechenden Schneckenparameter ist sichergestellt, dass jedes Formwerkzeug 4 bzw. der entsprechende Laufwagen 4' von der Schneckenwelle eingezogen werden kann, sobald es/er den Searbeitungsbereich der Formstation erreicht.

Die zweite Distanzhalteeinrichtung 4d' ist in Richtung des Doppelpfeils V einstellbar ausgebildet, beispielsweise durch Einschrauben bzw. Ausschrauben bezüglich des Grundkörpers 4a', durch das Einfügen von Distanzstücken (Distanzscheiben), mittels eines Rast-/Klemmmechanismus oder dergleichen. Auf den Nutzen dieser Konstruktion wird weiter unten anhand von Figur 5 noch genauer eingegangen.

Zunächst zeigt Figur 4 drei Laufwagen 4', welche im Wesentlichen den Laufwagen gemäß Figur 2 und Figur 3 entsprechen, beim Durchfahren eines gekrümmten Abschnitts 5.2 der Werkzeugsführungsstruktur 5. Gegenüber der Ausgestaltung in Figur 2 oder Figur 3 fällt auf, dass die zweite Distanzhalteeinrichtung 4d' stark verkürzt wurde, indem der gesamte abgerundete Endbereich entfernt wurde. Dies hat zur Folge, dass auch bei Kurvenfahrt, bei der sich die dritte Distanzhalteeinrichtung 4e' eines jeden Laufwagens 4' auf der Innenseite des gekrümmten Bahnverlaufs deutlich dem jeweils nachfolgenden laufwagen 4' im Bereich der zweiten Distanzhalteeinrichtung 4d' annähert, zu keiner Berührung zwischen den Laufwagen 4' in diesem Bereich kommt. Vielmehr erfolgt der Anschub weiterhin nur über die erste Distanzhalteeinrichtung 4c', wobei es aufgrund des angesprochenen schrägen Verkaufs der dritten Distanzhalteeinrichtung 4e' zu keiner Berührung oder Verkantung zwischen den einzelnen Laufwagen 4' auf der Bahninnerseite kommt. Der effektive Abstand zwischen benachbarten Laufwagen 4' entspricht gemäß Figur 4 dem Maß Y.

In der nachfolgenden Figur 5 beträgt das genannte Maß Y', wobei gilt, dass Y'>Y. Dies wird gemäß Darstellung in Figur 5 durch den gezielten Einsatz der zweiten Distanzhalteeinrichtung 4d' erreicht, worauf nachfolgend genauer eingegangen wird:
Gemäß der Darstellung in Figur 5 sind die zweiten Distanzhalteeinrichtungen 4d' durch Vorsehen bzw. Einstellen der vorne abgerundeten freien Endstrukturen derart verlängert, dass bei Kurvenfahrt nicht mehr die erste Distanzhalteeinrichtung 4c', sondern jeweils die zweite Distanzhalteeinrichtung 4d' eines nachfolgenden Laufwagens 4' mit dem vorausfahrenden Laufwagen 4' im Bereich von dessen dritter Distanzhalteeinrichtung in Anlage tritt. Zeitlich gesprochen, berühren sich die Laufwagen 4' demnach früher, so dass betragsmäßig ein größerer effektiver Abstand Y' zwischen aufeinander folgenden Laufwagen 4' existiert.
Wie der Fachmann erkennt, ist dieses größere Abstandsmaß Y' jedoch nur in den gekrümmten Bereichen 5.2 der Formstation wirksam. In den geraden Bereichen 5.1 gilt weiterhin der Abstand FL - X gemäß Figur 2, welcher im Wesentlichen durch die erste Distanzhalteeinrichtung 4c' vermittelt wird. Während nach dem vorliegenden Ausführungsbeispiel der letztgenannte Abstand FL - X über die festen Abmessungen der ersten Distanzhalteeinrichtung 4c' und der dritten Distanzhalteeinrichtung 4e' für einen gegebenen Laufwagen 4' fest vorgegeben ist. ergibt sich aufgrund der Einstellbarkeit im Bereich der zweiten Distanzhalteeinrichtung 4d' ein variables Abstandsmaß Y bzw. Y' in den gekrümmten Abschnitten 5.2, woraus insgesamt eine Einstellbarkeit der wirksamen Gesamtlänge der Formwerkzeuganordnung in einer Formstation mit gegebener Länge der Werkzeugführungsstruktur resultiert. Mit anderen Worten: Aufgrund der Einstellbarkeit der zweiten Distanzhalteeinrichtung 4d' kann ein Verwender der Formstation Einfluss darauf nehmen, ob mit einem gegebenen Formwerkzeug 4 bzw.
einen gegebenen Laufwagen 4' eine lückenlose Befüllung der Formstation möglich ist oder nicht bzw. wie viele Formwerkzeuge/Laufwagen hierfür notwendig sind. Somit ist eine gegebene Formstation bzw. Werkzeugsführungsstruktur auch bei unterschiedlichen Formatlängen verwendbar, ohne dass hierfür ein umfassender Umbau der Formstation bzw. der Werkzeugführungsstruktur erforderlich wäre.

Um den Betrieb der Formstation insbesondere hinsichtlich Gerauschentwicklung und Verschleiß günstig zu beeinflussen, kann weiterhin vorgesehen sein, dass im Bereich der ersten bis dritten Distanzhalteeinrichtungen 4c'-4e' geeignete Puffer-, Dämpfungs- oder Federmittel vorgesehen sind, beispielsweise auch in Form von Beschichtungen aus einem Elastomermaterial, ohne dass die Erfindung auf eine der vorliegenden Ausgestaltung beschränkt wäre. Außerdem ist es grundsätzlich möglich, zusätzlich oder alternativ zu der Einstellbarkeit der zweiten Distanzhalteeinrichtung 4d' auch die erste Distanzhalteeinrichtung 4c' und/oder die dritte Distanzhalteeinrichtung 4e' einstellbar auszubilden, um den Einsatzbereich der erfindungsgemäßen Formstation noch weiter zu vergrößern.

Um Formwerkzeuge mit größerer Formatlänge einsetzen zu können, wird der Fachmann zunächst eine geeignete formatabhängige Schneckenwelle oder eine sonstige geeignete Antriebseinrichtung vorsehen. Anschließend wird die Formstation 1 mit den geeigneten Formwerkzeugen 4 und den zugehörigen Laufwagen 4' bestückt. In der Regel wird dabei für eine vorgegebene Länge der geschlossenen Werkzeugsführungsstruktur keine tatsächlich lückenlose Auffüllung derselben erreichbar sein, was jedoch Voraussetzung für den vorgesehenen Schubantrieb ist. Der Fachmann wird dann durch geeignetes Einstellen insbesondere der zweiten Distanzhalteeinrichtung 4d' eines jeden Laufwagens 4' dafür sorgen, dass in den gekrümmten Abschnitten 5.2 der Werkzeugsführungsstruktur 5 der erforderliche Längenausgleich erfolgt, so dass dann zumindest vom Auslauf bis zum Einlauf der Schneckenwelle die Formwerkzeuge 4 bzw. die zugehörigen Laufwagen 4' für eine lückenlose Auffüllung der Werkzeugführungsstruktur sorgen und sich dabei zum Übermitteln der Anschubkräfte direkt berühren. Dadurch ist ein Umbau der Formstation im Bereich der Werkzeugführungsstruktur bei einem Formatwechsel nicht erforderlich.

Abschließend zeigt Figur 6 noch einmal detailliert anhand einer perspektivischen Ansicht eine mögliche Ausgestaltung des Laufwagens 4' mit Grundkörper 4a', Werkzeugbefestigungsstruktur 4b' sowie den ersten bis dritten Distanzhalteeinrichtungen 4c'-4e'. Auf der innenseite des Laufwagens 4' ist bei Bezugszeichen 4.6 noch eine weitere Rolle dargestellt, welche dazu vorgesehen ist, gemäß der Darstellung in Figur 2 der DE 10 2007 030 008 mit derjenigen Zwangsführungsstruktur im Bearbeitungsbereich zusammen zuwirken, welche für die Vertikalbewegung des Grundkörpers 4a' mit Werkzeugsbefestigungsstruktur 4b' bzw. des eigentlichen Formgebungswerkzeugs sorgt.

## Patentansprüche

1. Formstation (1) zur Herstellung von Formkörpern mit einer Anzahl von Formwerkzeugen (4), welche Formwerkzeuge dazu ausgebildet sind, zum Formen des Formkörpers aus mindestens einer Materialbahn (3) mit einer Fördereinrichtung (2) für die Materialbahn (3) zusammenzuwirken, in welcher Formstation die Formwerkzeuge (4) zumindest während einer zum Formen des Formkörpers benötigten Einwirkzeit in einem Bearbeitungsbereich synchron zusammen mit der Materialbahn (3) bewegbar und dabei in wenigstens einer ersten geschlossenen Werkzeugführungsstruktur (5) geführt sind, wobei die Werkzeugführungsstruktur (5) zumindest in dem Bearbeitungsbereich einen geraden Abschnitt (5.1) aufweist und dort im Wesentlichen parallel zur Materialbahn verläuft,
**dadurch gekennzeichnet, dass**
zum Bewegen der Formwerkzeuge (4) wenigstens eine Antriebseinrichtung (6) vorgesehen ist, welche dazu ausgebildet ist, auf wenigstens eines der Formwerkzeuge (4) einzuwirken, wobei die Formwerkzeuge (4) in der Werkzeugführungsstruktur (5) im Wesentlichen lückenlos angeordnet sind und schiebend aufeinander einwirken.

2. Formstation (1) nach Anspruch 1, **dadurch gekennzeichnet, dass** die Werkzeugführungsstruktur (5) neben geraden Abschnitten (5.1) auch gekrümmte Abschnitte (5.2) aufweist, wobei insbesondere die gekrümmten Abschnitte halbkreisförmig ausgebildet und die geraden Abschnitte zwischen den halbkreisförmigen Abschnitten angeordnet sind.

3. Formstation (1) nach mindestens einem der vorhergehenden Ansprüche,
**dadurch gekennzeichnet, dass** die Antriebseinrichtung wenigstens eine insbesondere synchron zu der Bewegung der Materialbahn (3) rotierende Schneckenwelle (6) aufweist, die zum Bewegen des wenigstens einen Formwerkzeugs (4) während der Einwirkzeit mit diesem zusammenwirkt.

4. Formstation (1) nach mindestens einem der vorhergehenden Ansprüche,
**dadurch gekennzeichnet, dass** wenigstens eines der Formwerkzeuge (4) eine erste Distanzhalteeinrichtung (4c') aufweist, welche einen ersten minimalen Abstand (FL-X; Y) zu einem benachbarten Formwerkzeug in der Werkzeugführungsstruktur (5) definiert.

5. Formstation (1) nach Anspruch 4, **dadurch gekennzeichnet, dass** die erste Distanzhalteeinrichtung (4c') dazu ausgebildet ist, bei im Wesentlichen geradliniger Führung des Formwerkzeugs (4) in der Werkzeugführungsstruktur (5) mit einem entsprechenden ersten Anlagebereich (4e') an dem benachbarten Formwerkzeug zusammenzuwirken.

6. Formstation (1) nach Anspruch 4 oder 5, **dadurch gekennzeichnet, dass** das wenigstens eine Formwerkzeug (4) eine zweite Distanzhalteeinrichtung (4d') aufweist, welche einen zweiten minimalen Abstand (Y') zu einem benachbarten Formwerkzeug in der Werkzeugführungsstruklur (5) definiert.

7. Formstation (1) nach Anspruch 6, **dadurch gekennzeichnet, dass** die zweite Distanzhalteeinrichtung (4d') dazu ausgebildet ist, bei krummliniger Führung des Formwerkzeugs (4) in der Werkzeugführungsstruktur (5) mit einem entsprechenden zweiten Anlagebereich (4e') an dem benachbarten Formwerkzeug zusammenzuwirken.

8. Formstation (1) nach einem der Ansprüche 4 und 5 und nach einem der Ansprüche 6 und 7, **dadurch gekennzeichnet, dass** der zweite Abstand (Y') betragsmäßig von dem ersten Abstand (Y) abweicht und insbesondere größer ist als der erste Abstand (Y).

9. Formstation (1) nach mindestens einem der Ansprüche 6 bis 8, **dadurch gekennzeichnet, dass** der erste Abstand (FL-X; Y) und/oder der zweite Abstand (Y') einstellbar ist.

10. Formstation (1) nach Anspruch 9, **dadurch gekennzeichnet, dass** durch die Einsteillbarkeit des ersten Abstands (Y) und/oder des zweiten Abstands (Y') die im Wesentlichen lückenlose Anordnung der Formwerkzeuge (4) in der Werkzeugführungsstruktur (5) auch bei Änderung einer Formatlänge (FL) der Formkörper und/oder bei Änderung einer Formwerkzeug-Abmessung realisierbar ist.

11. Formstation (1) nach zumindest Anspruch 4 oder 5, **dadurch gekennzeichnet, dass** der erste Abstand (FL-X) im Wesentlichen einer Formatlänge der Formkörper entspricht, wobei vorzugsweise der erste Abstand (FL-X) ein relativ geringes Mindermaß (X) gegenüber der Formatlänge aufweist.

12. Formstation (1) nach mindestens einem der Ansprüche 4 bis 11, **dadurch gekennzeichnet, dass** die erste Distanzhaiteeinrichtung (4c') und/oder die zweite Distanzhaiteeinrichtung (4d') und/oder eine zusätzliche dritte Distanzhalterung (4e') elastisch-nachgiebig ausgebildet ist, vorzugsweise durch Vorsehen von Federmitteln, Puffermitteln, Dämpfungsmitteln oder dergleichen.

13. Formstation (1) nach mindestens einem der Ansprüche 4 bis 12, **dadurch gekennzeichnet, dass** die erste Distanzhalteeinrichtung (4c') und/oder die zweite Distanzhaiteeinrichtung (4d') und/oder eine zusätzliche dritte Distanzhalterung (4e') an ihrem jeweiligen freien Ende, welches für das Zusammenwirken mit einem benachbarten Formwerkzeug vorgesehen ist, eine Abrundung aufweist.

14. Verwendung der Formstation (1) nach einem der vorhergehenden Ansprüche zum Herstellen von Formkörpern, insbesondere Stilleinlagen, aus einer Materialbahn (3).

15. Verwendung nach Anspruch 14, **dadurch gekennzeichnet, dass** die Materialbahn (3) ein zusammengesetztes Material mit wenigstens einer saugfähigen, insbesondere Granulat angereicherten Schicht sowie weiteren Fluid dichten oder Fluid durchlässigen Schichten ist.
